# EUROPÄISCHE PATENTSCHRIFT

(11) **EP 0 599 928 B1**
(45) Veröffentlichungstag und Bekanntmachung des Hinweises auf die Patenterteilung: **19.03.1997**
(21) Anmeldenummer: 92917608.9
(22) Anmeldetag: 19.08.1992
(51) Int. Cl.: C09K 19/58, G02F 1/1337, C07D 273/00, C07D 285/00, C07D 291/02, C07D 257/02, C07D 259/00, C07D 273/01

(54) **STABILISIERTE KOMPLEXLIGANDEN UND DEREN VERWENDUNG IN FLÜSSIGKRISTALLDISPLAYS**
STABILIZED COMPLEX LIGANDS AND THEIR USE IN LIQUID CRYSTAL DISPLAYS
LIGANDS COMPLEXES STABILISES ET LEUR UTILISATION DANS DES AFFICHAGES A CRISTAUX LIQUIDES

(30) Priorität: 21.08.1991 DE 4127658
(43) Veröffentlichungstag der Anmeldung: 08.06.1994
(73) Patentinhaber: HOECHST AKTIENGESELLSCHAFT, 65926 Frankfurt am Main (DE)
(72) Erfinder: MANERO, Javier, D-6230 Frankfurt am Main 80 (DE); RÖSCH, Norbert, D-6000 Frankfurt am Main (DE)
(86) Internationale Anmeldenummer: EP9201895
(87) Internationale Veröffentlichungsnummer: WO9304142

(56) Entgegenhaltungen:
- EP-A- 0 429 662
- WO-A-91/08272
- DE-A- 1 953 249
- FR-A- 2 246 555
- US-A- 4 267 256
- PATENT ABSTRACTS OF JAPAN vol. 3, no. 113 19. September 1977

## Beschreibung

In WO 91/ 08272 wird die Verwendung von Komplexliganden in ferroelektrischen Flüssigkristallmischungen (FLC) bzw. in Orientierungsschichten von Flüssigkristall Displays beschrieben. Mit diesen Stoffen kann das Orientierungs- oder Schaltverhalten der FLC-Mischungen derart beeinflußt werden, daß sich Kontrast und Helligkeit in den Displays erhöhen und die Bildung von Geisterbildern unterdrückt wird.

Es hat sich jedoch im Laufe der Zeit gezeigt, daß sich insbesondere stickstoffhaltige, Komplexliganden unter Einfluß von Licht (UV-Licht) und Wärme zersetzen. Die Zersetzungsprodukte zeichnen sich durch eine gelbe Farbe aus. Werden die stickstoffhaltigen Komplexliganden in Flüssigkristallen gelöst, so führt die geringe Stabilität der Komplexliganden schließlich zu einer Gelbfärbung der Flüssigkristall-Mischung. Ist die Zersetzung des Komplexliganden weit fortgeschritten, so kann letzlich auch die positive Wirkung der Liganden verlorengehen.

Überraschend wurde nun gefunden, daß man diese Stoffe stabilisieren kann, indem sie durch sperrige sekundäre oder tertiäre Carbon- oder Sulfonsäuren derivatisiert werden. Derart derivatisierte Verbindungen zeigen eine gleich gute Wirksamkeit, z. B. auf Unterdrückung von Geisterbildern, wie die nicht derivatisierte Verbindung, und sie haben den Vorteil, daß die Zersetzung und damit eine Gelbfärbung der Flüssigkristallmischung nicht mehr auftritt.

Die Erfindung betrifft somit eine Verbindung der allgemeinen Formel I, 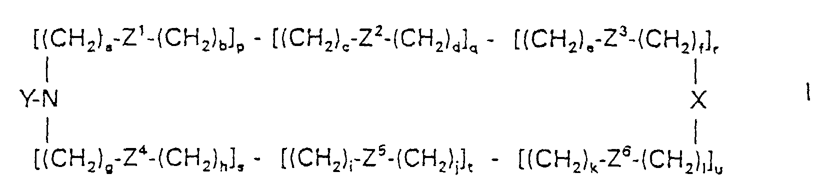 in der
- Z¹, Z², Z³, Z⁴, Z⁵, Z⁶ gleich oder verschieden die Gruppe -O-, -S- oder eine Einfachbindung bedeuten, wobei aber mindestens 3 der Z-Gruppen in der Formel I enthalten sein müssen;
- Y die Gruppe B-(CH₂)ₘ-A- bedeutet, in der
   A: 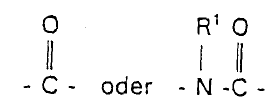 ist,
   B die Gruppe 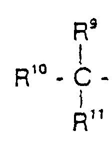 bedeutet,
   wobei R¹ C₁-C₈ Alkyl oder Phenyl ist, das durch 1 bis 3 C₁-C₃-Alkyl- oder Alkoxygruppen substituiert sein kann, und
   R⁹, R¹⁰, R¹¹ gleich oder verschieden Alkyl verzweigt oder unverzweigt mit 1 bis 6 C-Atomen bedeuten oder zusammen mit dem tragenden C-Atom ein cyclisches oder polycyclisches System mit 2 bis 12 Ringen bilden;
- m die Zahl 0 oder 1 ist,
- X N-Y, -O- oder Z¹ bis Z⁶ ist,
- a, b, c, d, e, f, g, h, i, j, k, l eine Zahl von 0 bis 3 ist, wobei die Summe aus a + b + c + d + e + f + g + h + i + j + k + l 8 bis 16 C-Atomen entspricht, mit der Maßgabe, daß a, f, g und/oder l nicht Null sein dürfen und daß Z¹, Z², Z³ sowie Z⁴, Z⁵, Z⁶ nicht direkt benachbart sind; und
- p, q, r, s, t, u 0 oder 1 sind, wobei die Summe von p, q, r, s, t, u 2 bis 6 beträgt;
a, b, c, d, e, f, g, h, i, j, k, l und p, q, r, s, t, u sind so zu wählen, daß sich Ringgrößen von 12 bis 24 ergeben.

Bevorzugt sind Verbindungen der Formell, in der Y die folgenden Gruppen darstellt:
Adamantylcarbonyl 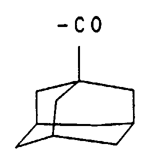
Pivaloyl 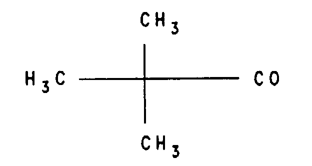
Mesitylencarbonyl 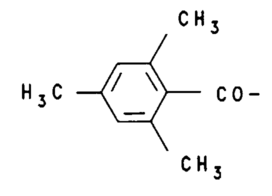
2,6 Dimethylbenzoyl 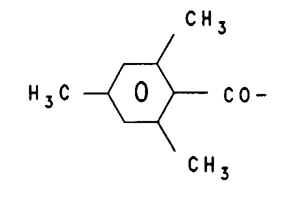
Mesitylensulfonyl 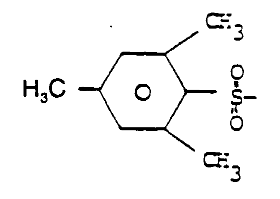
Cubancarbonyl 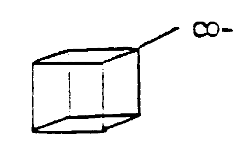
[2.2.2.]-Bicyclooctan-1-carbonyl 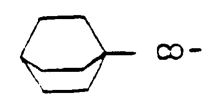
Adamantylacetyl 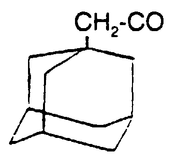
Adamantylthiouryl 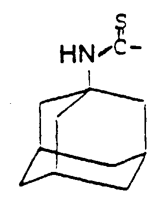
Noradamantylcarbonyl 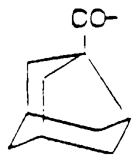
1-Norbornancarbonyl 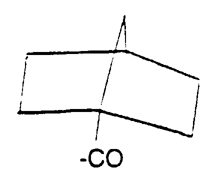
Pagodancarbonyl 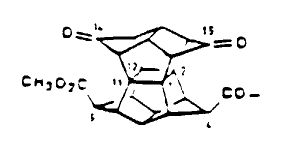
3.3-Dimethylbutyroyl 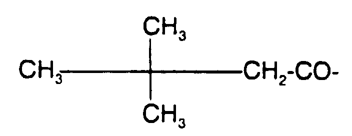
2.2-Dimethylbutyroyl 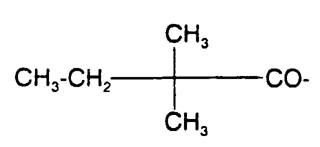
ganz besonders bevorzugt sind Verbindungen der Formel I, in der Y die folgenden Gruppen darstellt:
Adamantylcarbonyl
Pivaloyl
Adamantylacetyl
2,2-Dimethylbutyroyl
3,3-Dimethylbutyroyl
1-Noradamantylcarbonyl

Die Herstellung der Verbindungen der Formel I kann nach den im folgenden zitierten Methoden erfolgen:
1) Houben-Weyl, Methoden der Organischen Chemie, 4. Aufl., Bd. VIII, Sauerstoffverbindungen III, S. 653 bis 671
2) Houben-Weyl, Methoden der Organischen Chemie, 4. Aufl., Bd. E4, Kohlensäurederivate, S. 484 bis 505
3) Houben-Weyl, Methoden der Organischen Chemie, 4. Aufl., Bd. El, Phosphorverbindungen I, S. 271 bis 313
4) Houben-Weyl, Methoden der Organischen Chemie, 4. Aufl., Bd. El, Phosphorverbindungen I, S. 313 bis 488
5) Houben-Weyl, Methoden der Organischen Chemie, 4. Aufl., Bd. E2, Phosphorverbindungen II, S. 394 bis 398
6) Houben-Weyl, Methoden der Organischen Chemie, 4. Aufl., Bd. E2, Phosphorverbindungen II, S. 487 bis 831
7) Houben-Weyl, Methoden der Organischen Chemie, 4. Aufl., Bd. E11, Organische Schwefelverbindungen I, S. 655 bis 662
8) Houben-Weyl, Methoden der Organischen Chemie, 4. Aufl., Bd. E11, Organische Schwefelverbindungen II, S. 1.098 bis 1.103

Die Verbindung der Formel I kann ferroelektrischen Flüssigkristallmischungen zugesetzt sowie in oder auf Orientierungsschichten gebracht werden. Die Bindung an die Orientierungsschicht kann über chemische Ankopplung, d. h. Kovalenzbindungen, bzw. durch Physisorption, d. h. durch intermolekulare Anziehungskräfte, an die Orientierungsschichtmoleküle erfolgen. Bei der Physisorption kann die Stärke der Ankopplung an die Orientierungsschichtmoleküle durch Verwendung möglichst polarer Verbindungen der Formel I erhöht werden.

Die Verbindung der allgemeinen Formel I wird in Konzentrationen von 0,01 bis 20 Gew.-%, bevorzugt 0,1 bis 5 Gew.-%, in die Flüssigkristallmischung ein - bzw. in oder auf die Orientierungsschicht aufgebracht.

Die folgenden Beispiele dienen der weitergehenden Erläuterung der Erfindung.

### Beispiele

### Beispiel 1: Synthese von Carbonsäurehalogeniden aus Carbonsäuren

15 ml Carbonsäure werden in 30 bis 100 ml Benzol gelöst und mit 12 ml Oxalylchlorid sowie 2 Tropfen Pyridin versetzt. Nach 18 Std. wird das Solvens abgezogen. Die Probe wird 2 x mit 12 ml Benzol versetzt und nach erneutem Abziehen des Solvens an der Ölpumpe getrocknet.

### Beispiel 2: 4,10-Bis(tert.-butylcarbonyl)-1,7-dioxa-4,10-diazacyclododecan

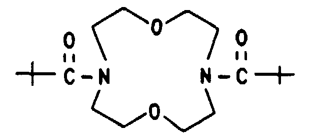

3,81 mmol 1,7-Dioxa-4,10-diazacyclododecan werden in 50 ml CH₂Cl₂ gelöst. Man versetzt mit 1,2 ml Triethylamin und gibt noch 0,1 g DMAP (=4-Dimethylaminopyridin) als Katalysator hinzu. Anschließend fügt man 7,64 mmol Pivalinsäurechlorid zu. Es wird 18 Stunden bei Raumtemparatur gerührt.

Die Reaktionslösung wird 2x mit 1n HCl, anschließend 2x mit gesättigter NaHCO₃-Lösung gewaschen. Man trocknet über MgSO₄ und zieht das Solvens ab. Das Rohprodukt wird säulenchromatographisch an Kieselgel mit CH₂Cl₂/Methanol = 20/1 gereinigt.

### Beispiel 3: 4,10-Bis(1-Adamantylcarbonyl)-1,7-dioxa-4,10-diazacyclododecan

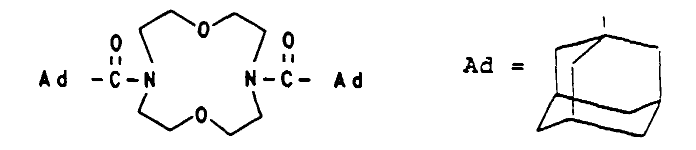

Analog Beispiel 2 aus 1,7-Dioxa-4,10-diazacyclododecan und 1-Adamantylcarbonsäurechlorid.

### Beispiel 4: 4,10-Bis((1-adamantyl)acetyl)-1,7-dioxa-4,10-diazacyclododecan

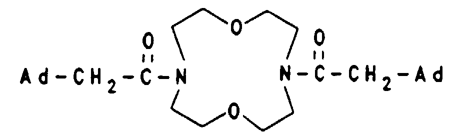

Analog Beispiel 2 aus 1,7-Dioxa-4,10-diazacyclododecan und (1-Adamantyl)essigsäurechlorid.

### Beispiel 5: 7,13-Bis(tert.-butylcarbonyl)-1,4,10-trioxa-7,13-diazacyclopentadecan

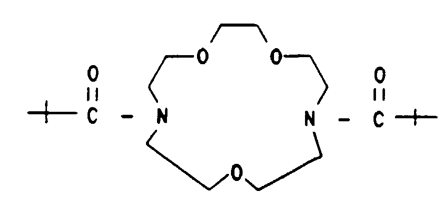

Analog Beispiel 2 aus 1,4,10-Trioxa-7,13-diazacyclopentadecan und Pivalinsäurechlorid.

### Beispiel 6: 7,13-Bis(1-Adamantylcarbonyl)-1,4,10-trioxa-7,13-diazacyclopentadecan

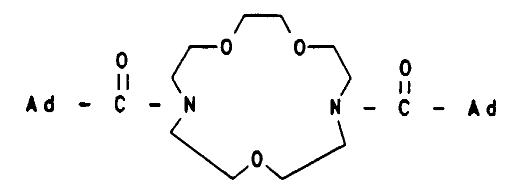

Analog Beispiel 2 aus 1,4,10-Trioxa-7,13-diazacyclopentadecan und 1-Adamantylcarbonsäurechlorid.

### Beispiel 7: 7,1 3-Bis((1-adamantyl)acetyl)-1,4,10-trioxa-7,13-diazacyclopentadecan

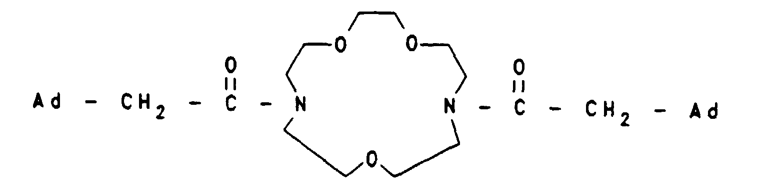

Analog Beispiel 2 aus 1,4,10-Trioxa-7,13-diazacyclopentadecan und (1-Adamantyl)essigsäurechlorid.

### Beispiel 8: 4,13-Bis(tert.-butylcarbonyl)-1,7,10,16-tetraoxa-4,13-diazacyclooctadecan

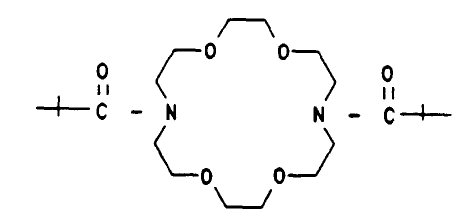

Analog Beispiel 2 aus 1,7,10,16-Tetraoxa-4,13-diazacyclooctadecan und Pivalinsäurechlorid.

### Beispiel 9: 4,13-Bis(1-Adamantylcarbonyl)-1,7,10,16-tetraoxa-4,13-diazacyclooctadecan

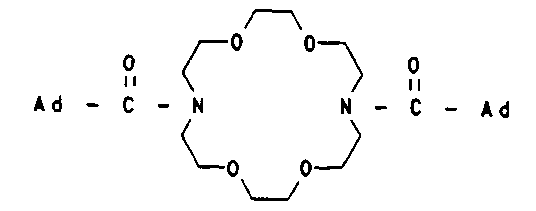

Analog Beispiel 2 aus 1,7,10,16-Tetraoxa-4,13-diazacyclooctadecan und 1-Adamantylcarbonsäurechlorid.

### Beispiel 10: 4,13-Bis((1-adamantyl)acetyl)-1,7,10,16-tetraoxa-4,13-diazacyclooctadecan

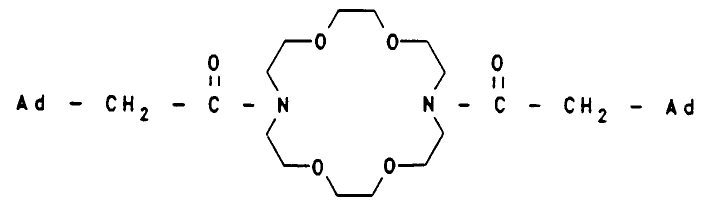

Analog Beispiel 2 aus 1,7,10,16-Tetraoxa-4,13-diazacyclooctadecan und (1-Adamantyl)essigsäurechlorid.

### Beispiel 11: 4,16-Bis(tert.-butylcarbonyl)-1,7,10,13,19-pentaoxa-4,16-diazacycloheneicosan

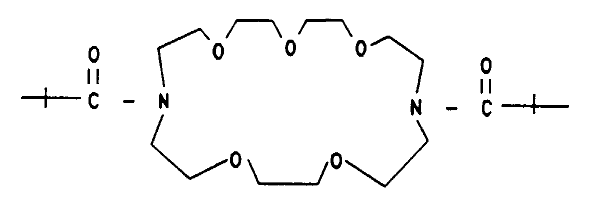

Analog Beispiel 2 aus 1,7,10,13,19-Pentaoxa-4,16-diazacycloheneicosan und Pivalinsäurechlorid.

### Beispiel 12: 4,16-Bis(1-Adamantylcarbonyl)-1,7,10,13,19-pentaoxa-4,16-diazacycloheneicosan

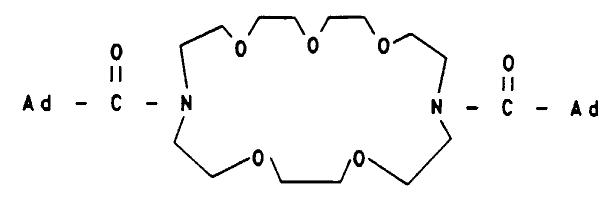

Analog Beispiel 2 aus 1,7,10,13,1 9-Pentaoxa-4,16-diazacycloheneicosan und 1-Adamantylcarbonsäurechlorid.

### Beispiel 13: 4,16-Bis((1-adamantyl)acetyl)-1,7,10,13,19-pentaoxa-4,16-diazacycloheneicosan

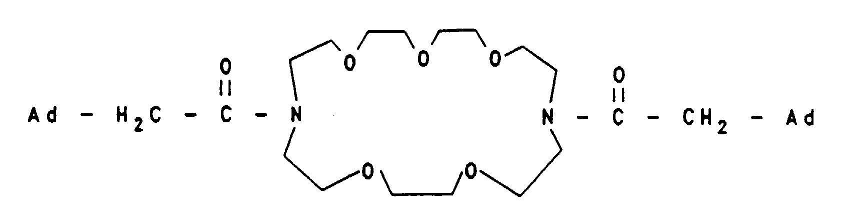

Analog Beispiel 2 aus 1,7,10,13,19-Pentaoxa-4,16-diazacydoheneicosan und (1-Adamantyl)essigsäurechlorid.

### Beispiel 14: 1-(Tert.-butylcarbonyl)-1-aza-4,7,10-trioxacyclododecan

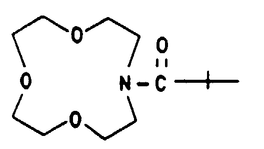

3,81 mmol 1-Aza-4,7,10-trioxacyclododecan werden in 50 ml CH₂Cl₂ gelöst. Man versetzt mit 0,6 ml Triethylamin und gibt noch 0,1 g DMAP (=4-Dimethylaminopyridin) als Katalysator hinzu. Anschließend fügt man 3,82 mmol Pivalinsäurechlorid zu. Es wird 18 Stunden bei Raumtemparatur gerührt.

Die Reaktionslösung wird 2x mit 1n HCl, anschließend 2x mit gesättigter NaHCO₃-Lösung gewaschen. Man trocknet über MgSO₄ und zieht das Solvens ab. Das Rohprodukt wird säulenchromatographisch an Kieselgel mit CH₂Cl₂/Methanol=20/1 gereinigt.

### Beispiel 15: 1-(1-Adamantylcarbonyl)-1-aza-4,7,10-trioxacyclododecan

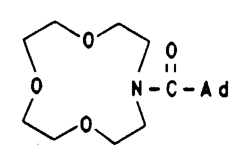

Analog Beispiel 14 aus 1-Aza-4,7,10-trioxacyclododecan und 1-Adamantylcarbonsäurechlorid.

### Beispiel 16: 1-((1-Adamantyl)acetyl)-1-aza-4,7,10-trioxacyclododecan

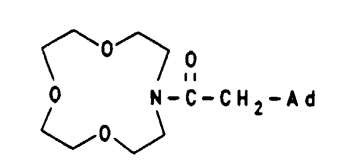

Analog Beispiel 14 aus 1-Aza-4,7,10-trioxacyclododecan und (1-Adamantyl)essigsäurechlorid.

### Beispiel 17: 1-(Tert.-butylcarbonyl)-1-aza-4,7,10,13-tetraoxacyclopentadecan

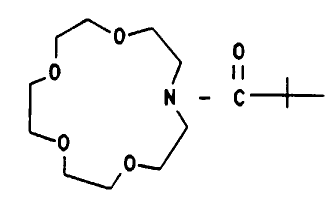

Analog Beispiel 14 aus 1-Aza-4,7,10,13-tetraoxacyclopentadecan und Pivalinsäurechlorid.

### Beispiel 18: 1-(1-Adamantylcarbonyl)-1-aza-4,7,10,13-tetraoxacyclopentadecan

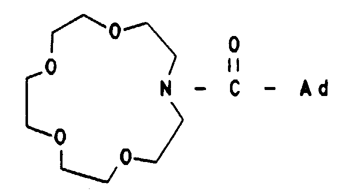

Analog Beispiel 14 aus 1-Aza-4,7,10,13-tetraoxacyclopentadecan und 1-Adamantylcarbonsäurechlorid.

### Beispiel 19: 1-((1-Adamantyl)acetyl)-1-aza-4,7,10,13-tetraoxacyclopentadecan

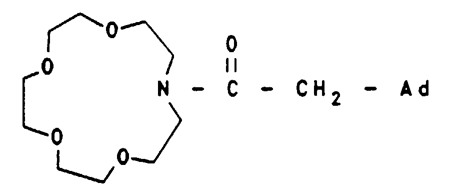

Analog Beispiel 14 aus 1-Aza-4,7,10,13-tetraoxacyclopentadecan und (1-Ada-mantyl)essigsäurechlorid.

### Beispiel 20: 1-(Tert.-butylcarbonyl)-1-aza-4,7,10,13,16-pentaoxacyclooctadecan

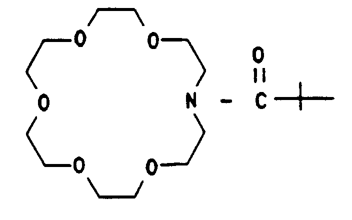

Analog Beispiel 14 aus 1-Aza-4,7,10,13,16-pentaoxacyclooctadecan und Pivalinsäurechlorid.

### Beispiel 21: 1-(1-Adamantylcarbonyl)-1-aza-4,7,10,13,16-pentaoxacyclooctadecan

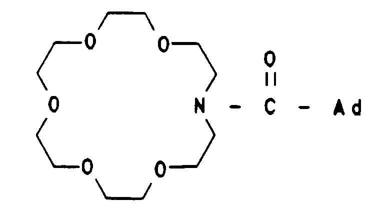

Analog Beispiel 14 aus 1-Aza-4,7,10,13,16-pentaoxacyclooctadecan und 1-Adamantylcarbonsäurechlorid.

### Beispiel 22: 1-((1-Adamantyl)acetyl)-1-aza-4,7,10,13,16-pentaoxacyclooctadecan

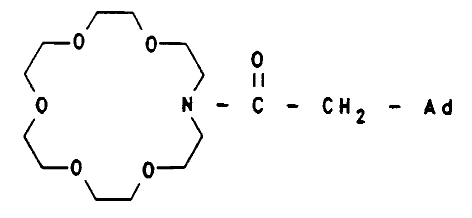

Analog Beispiel 14 aus 1-Aza-4,7,10,13,16-pentaoxacyclooctadecan und (1-Adamantyl)essigsäurechlorid.

### Beispiel 23: 1-(Tert.-butylcarbonyl)-1-aza-4,7,10,13,16,19-hexaoxacycloheneicosan

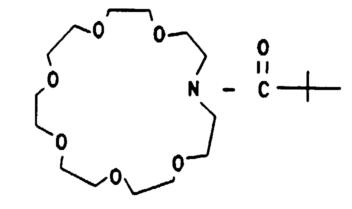

Analog Beispiel 14 aus 1-Aza-4,7,10,13,16,19-hexaoxacycloheneicosan und Pivalinsäurechlorid.

### Beispiel 24: 1-(1-Adamantylcarbonyl)-1-aza-4,7,10,13,16,19-hexaoxacycloheneicosan

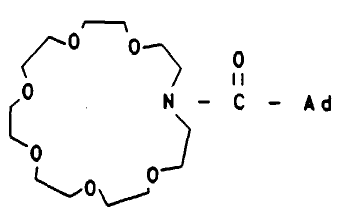

Analog Beispiel 14 aus 1-Aza-4,7,10,13,16,19-hexaoxacycloheneicosan und 1-Adamantylcarbonsäurechlorid.

### Beispiel 25: 1-((1-Adamantyl)acetyl)-1-aza-4,7,10,13,16,19-hexaoxacycloheneicosan

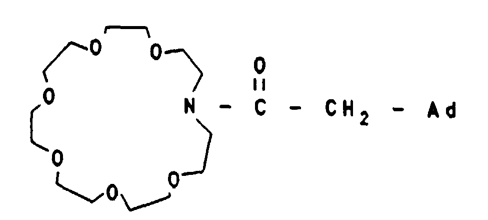

Analog Beispiel 14 aus 1-Aza-4,7,10,13,16,19-hexaoxacycloheneicosan und (1-Adamantyl)essigsäurechlorid.

### Beispiel 26: 4,10-Bis(2,2-dimethylbutyroyl)-1,7-dioxa-4,10-diazacyclododecan

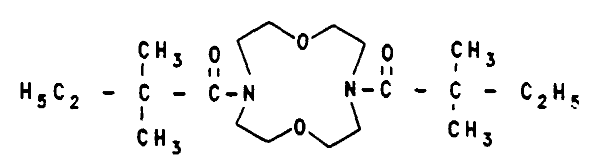

Analog Beispiel 2 aus 1,7-Dioxa-4,10-diazacyclododecan und 2,2-Dimethylbuttersäurechlorid.

### Beispiel 27: 7,13-Bis(2,2-dimethylbutyroyl)-1,4,10-trioxa-7,13-diazacyclopentadecan

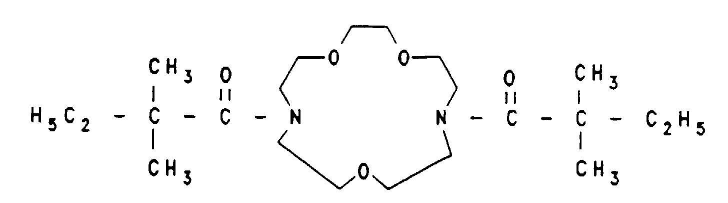

Analog Beispiel 2 aus 1,4,10-Trioxa-7,13-diazacyclopentadecan und 2,2-Dimethylbuttersäurechlorid.

### Beispiel 28: 4,13-Bis(2,2-dimethylbutyroyl)-1,7,10,16-tetraoxa-4,13-diazacyclooctadecan

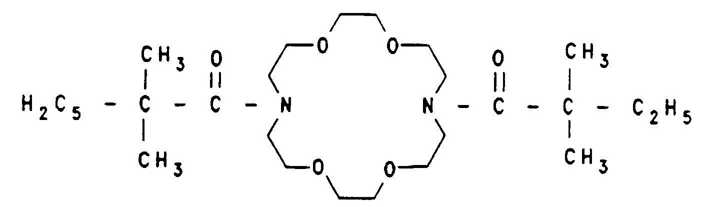

Analog Beispiel 2 aus 1,7,10,16-Tetraoxa-4,13-diazacyclooctadecan und 2,2-Dimethylbuttersäurechlorid.

### Beispiel 29: 4,1 6-Bis(2,2-dimethylbutyroyl)-1,7,10,13,19-pentaoxa-4,16-diazacycloheneicosan

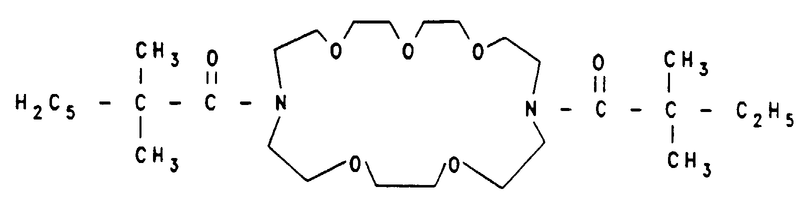

Analog Beispiel 2 aus 1,7,10,13,19-Pentaoxa-4,16-diazacycloheneicosan und 2,2-Dimethylbuttersäurechlorid.

### Beispiel 30: 1-(2,2-Dimethylbutyroyl)-1-aza-4,7,10-trioxacyclododecan

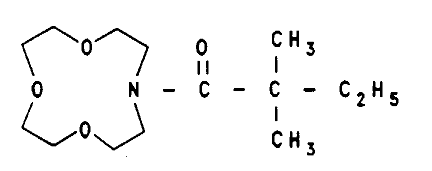

Analog Beispiel 14 aus 1-Aza-4,7,10-trioxacyclododecan und 2,2-Dimethylbuttersäurechlorid.

### Beispiel 31: 1-(2,2-Dimethylbutyroyl)-1-aza-4,7,10,13-tetraoxacyclopentadecan

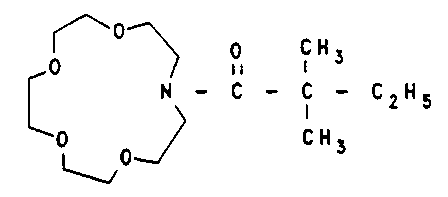

Analog Beispiel 14 aus 1-Aza-4,7,10,13-tetraoxacyclopentadecan und 2,2-Dimethylbuttersäurechlorid.

### Beispiel 32: 1-(2,2-Dimethylbutyroyl)-1-aza-4,7,10,13,16-pentaoxacyclooctadecan

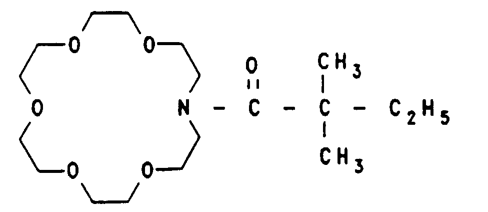

Analog Beispiel 14 aus 1-Aza-4,7,10,13,16-pentaoxacyclooctadecan und 2,2-Dimethylbuttersäurechlorid.

### Beispiel 33: 1-(2,2-Dimethylbutyroyl)-1-aza-4,7,10,13,16,19-hexaoxacycloheneicosan

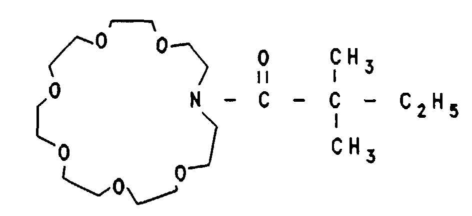

Analog Beispiel 14 aus 1-Aza-4,7,10,13,16,19-hexaoxacycloheneicosan und 2,2-Dimethylbuttersäurechlorid.

### Beispiel 34: 4,10-Bis(3,3-dimethylbutyroyl)-1,7-dioxa-4,10-diazacyclododecan

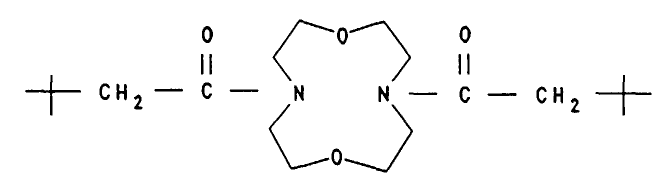

Analog Beispiel 2 aus 1,7-Dioxa-4,10-diazacyclododecan und 3,3-Dimethylbuttersäurechlorid.

### Beispiel 35: 7,13-Bis(3,3-dimethylbutyroyl)-1,4,10-trioxa-7,13-diazacyclopentadecan

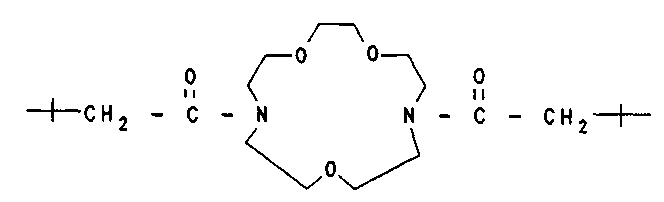

Analog Beispiel 2 aus 1,4,10-Trioxa-7,13-diazacyclopentadecan und 3,3-Dimethylbuttersäurechlorid.

### Beispiel 36: 4,13-Bis(3,3-dimethylbutyroyl)-1,7,10,16-tetraoxa-4,13-diazacyclooctadecan

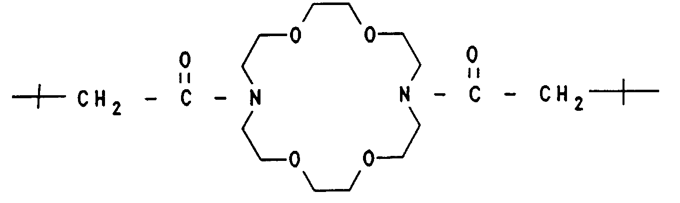

Analog Beispiel 2 aus 1,7,10,16-Tetraoxa-4,13-diazacyclooctadecan und 3, 3-Dimethylbuttersäurechlorid.

### Beispiel 37: 4,16-Bis(3,3-dimethylbutyroyl)-1,7,10,13,19-pentaoxa-4,16-diazacycloheneicosan

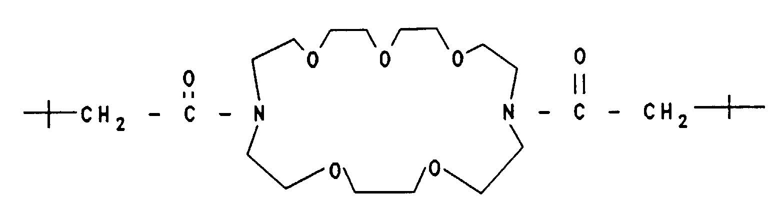

Analog Beispiel 2 aus 1,7,10,13,19-Pentaoxa-4,16-diazacycloheneicosan und 3,3-Dimethylbuttersäurechlorid.

### Beispiel 38: 1-(3,3-Dimethylbutyroyl)-1-aza-4,7,10-trioxacyclododecan

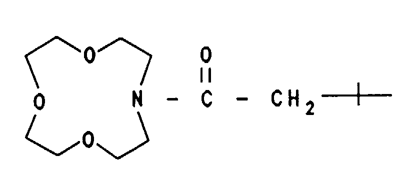

Analog Beispiel 14 aus 1-Aza-4,7,10-trioxacyclododecan und 3,3-Dimethylbuttersäurechlorid.

### Beispiel 39: 1-(3,3-Dimethylbutyroyl)-1-aza-4,7,10,13-tetraoxacyclopentadecan

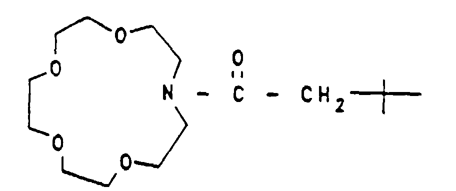

Analog Beispiel 14 aus 1-Aza-4,7,10,13-tetraoxacyclopentadecan und 3,3-Dimethylbuttersäurechlorid.

### Beispiel 40: 1-(3,3-Dimethylbutyroyl)-1-aza-4,7,10,13,16-pentaoxacyclooctadecan

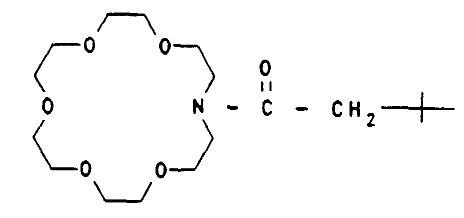

Analog Beispiel 14 aus 1-Aza-4,7,10,13,16-pentaoxacyclooctadecan und 3,3-Dimethylbuttersäurechlorid.

### Beispiel 41: 1-(3,3-Dimethylbutyroyl)-1-aza-4,7,10,13,16,19-hexaoxacycloheneicosan

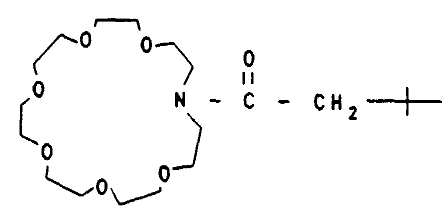

Analog Beispiel 14 aus 1-Aza-4,7,10,13,16,19-hexaoxacycloheneicosan und 3,3-Dimethylbuttersäurechlorid.

### Beispiel 42: 4,10-Bis(1-noradamantylcarbonyl)-1,7-dioxa-4,10-diazacyclododecan

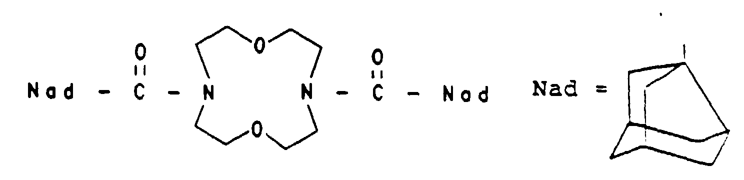

Analog Beispiel 2 aus 1,7-Dioxa-4,10-diazacyclododecan und 1-Noradamantancarbonsäurechlorid.

### Beispiel 43: 7,13-Bis(1-noradamantylcarbonyl)-1,4,10-trioxa-7,13-diazacyclopentadecan

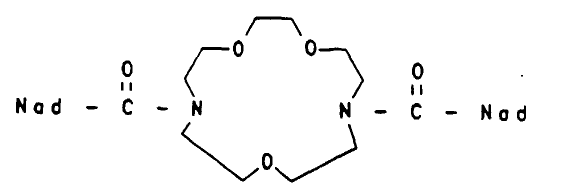

Analog Beispiel 2 aus 1,4,10-Trioxa-7,13-diazacyclopentadecan und 1-Noradamantancarbonsäurechlorid.

### Beispiel 44: 4,13-Bis(1-noradamantylcarbonyl)-1,7,10,16-tetraoxa-4,13-diazacyclooctadecan

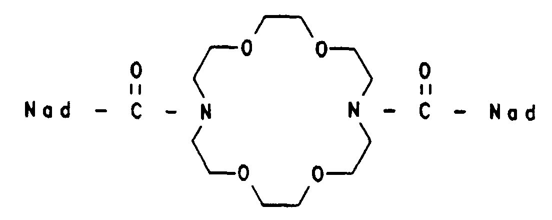

Analog Beispiel 2 aus 1,7,10,16-Tetraoxa-4,13-diazacyclooctadecan und 1-Noradamantancarbonsäurechlorid.

### Beispiel 45: 4,16-Bis(1-noradamantylcarbonyl)-1,7,10,13,19-pentaoxa-4,16-diazacycloheneicosan

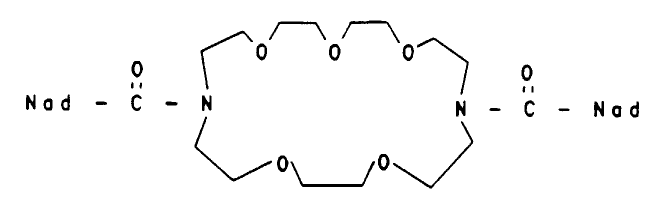

Analog Beispiel 2 aus 1,7,10,13,19-Pentaoxa-4,16-diazacycloheneicosan und 1-Noradamantancarbonsäurechlorid.

### Beispiel 46: 1-(1-Noradamantylcarbonyl)-1-aza-4,7,10-trioxacyclododecan

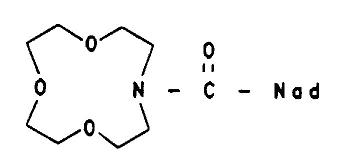

Analog Beispiel 14 aus 1-Aza-4,7,10-trioxacyclododecan und 1-Noradamantancarbonsäurechlorid.

### Beispiel 47: 1-(1-Noradamantylcarbonyl)-1-aza-4,7,10,13-tetraoxacyclopentadecan

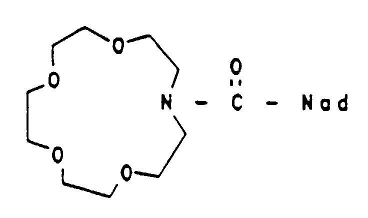

Analog Beispiel 14 aus 1-Aza-4,7,10,13-tetraoxacyclopentadecan und 1-Noradamantancarbonsäurechlorid.

### Beispiel 48: 1-(1-Noradamantylcarbonyl)-1-aza-4,7,10,13,16-pentaoxacyclooctadecan

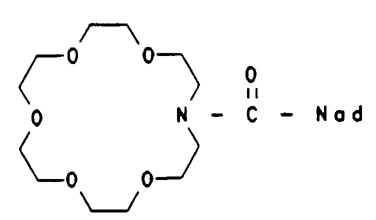

Analog Beispiel 14 aus 1-Aza-4,7,10,13,16-pentaoxacyclooctadecan und 1-Noradamantancarbonsäurechlorid.

### Beispiel 49: 1-(1-Noradamantylcarbonyl)-1-aza-4,7,10,13,16,19-hexaoxacycloheneicosan

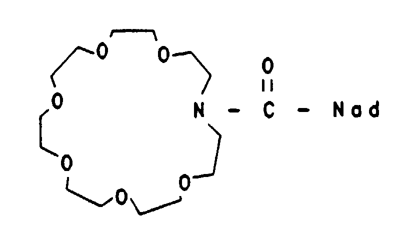

Analog Beispiel 14 aus 1-Aza-4,7,10,13,16,19-hexaoxacycloheneicosan und 1-Noradamantancarbonsäurechlorid.

### Beispiel 50: Anwendungsbeispiel - Stabilitätsnachweis

Zum Nachweis der erhöhten chemischen Stabilität der erfindungsgemäßen Verbindungen wurde Z1 und das als lichtempfindlich bekannte Kryptofix 22 in eine flüssigkristalline Mischung gegeben.

Beide Proben wurden bei 50°C mit einer UV-Lampe (Sun Tester der Firma Heraeus) 30 Min. bestrahlt. Anschließend wurden die bestrahlten Proben in Methylenchlorid gelöst und die entsprechenden optischen Spektren mit einem Photometer aufgenommen. Als Referenz diente der reine, in Methylenchlorid gelöste Flüssigkristall.
Z1: 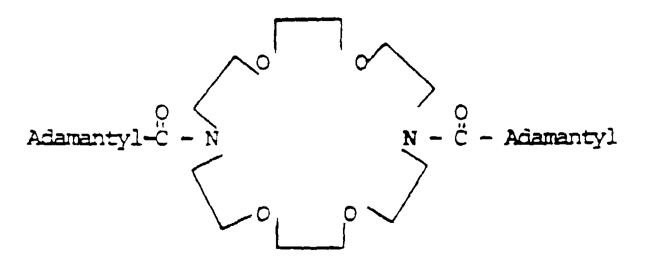
Kryptofix 22

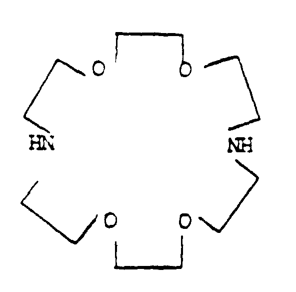

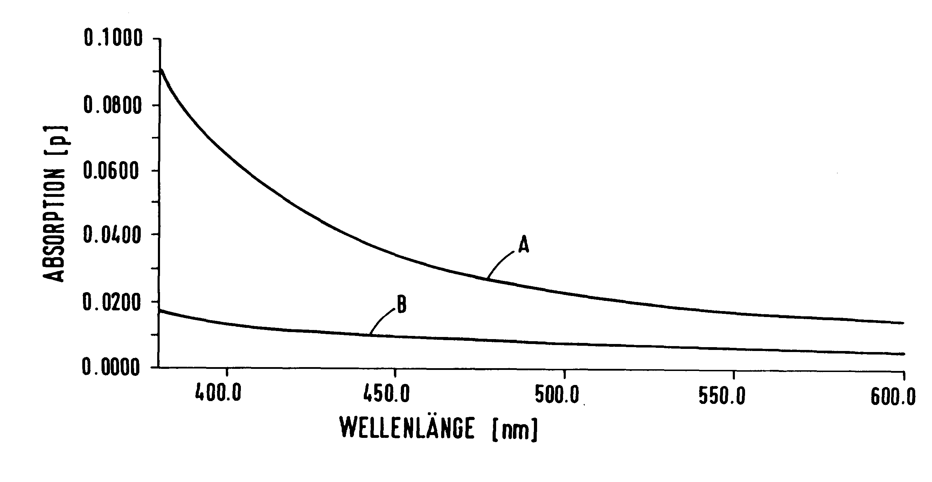

Die Abbildung zeigt die optischen Spektren der Flüssigkristallmischung mit Kryptofix 22 (Kurve A) und der Mischung mit Z1 (Kurve B). Die bei Zersetzung von Kryptofix 22 entstehende Gelbfärbung der Probe führt im Spektrum zu einer Absorption im Bereich zwischen 400 bis 500 nm. In diesem Bereich zeigt die Mischung mit der erfindungsgemäßen Verbindung keine nennenswerte Absorption.

## Patentansprüche

1. Verbindung der allgemeinen Formel I 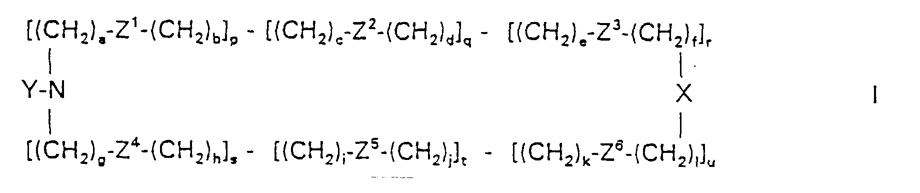 in der
- Z¹, Z², Z³, Z⁴, Z⁵, Z⁶ gleich oder verschieden die Gruppe -O-, -S-, oder eine Einfachbindung bedeuten, wobei aber mindestens 3 der Z-Gruppen in der Formel I enthalten sein müssen;
- Y die Gruppe B-(CH₂)ₘ-A- bedeutet, in der
A: 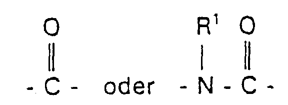 ist
B die Gruppe 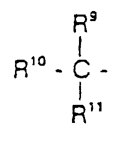 bedeutet,
wobei R¹ C₁-C₁₈ Alkyl oder Phenyl ist, das durch 1 bis 3 C₁-C₄-Alkyl- oder Alkoxygruppen substituiert sein kann, und R⁹, R¹⁰, R¹¹ gleich oder verschieden Alkyl verzweigt oder unverzweigt mit 1 bis 6 C-Atomen bedeuten oder zusammen mit dem tragenden C-Atom ein cyclisches oder polycyclisches System mit 2 bis 12 Ringen bilden;
- m die Zahl 0 oder 1 ist,
- X N-Y, -O- oder Z¹ bis Z⁶ ist,
- a, b, c, d, e, f, g, h, i, j, k, l eine Zahl von 0 bis 3 ist, wobei die Summe aus a + b + c + d + e + f + g + h + i + j + k + l 8 bis 16 C-Atomen entspricht, mit der Maßgabe, daß a, f, g und/oder l nicht Null sein dürfen, und daß Z¹, Z², Z³ sowie Z⁴, Z⁵, Z⁶ nicht direkt benachbart sind; und - p, q, r, s, t, u 0 oder 1 sind, wobei die Summe von p, q, r s, t, u 2 bis 6 beträgt;
a, b, c, d, e, f, g, h, i, j, k, l und p, q, r, s, t, u sind so zu wählen, daß sich Ringgrößen von 12 bis 24 ergeben.

2. Verfahren zur Stabilisierung von Komplexliganden, dadurch gekennzeichnet, daß die Komplexliganden mit sperrigen sekundären oder tertiären Carbonsäuren und/oder Sulfonsäuren derivatisiert werden.

3. Verwendung der Verbindungen der allgemeinen Formel I nach Anspruch 1 als Zusatz zu Flüssigkristallmischungen.

4. Verwendung der Verbindung der allgemeinen Formel I nach Anspruch 1 als Zusatz in Orientierungsschichten.

5. Verwendung nach Anspruch 3 oder 4 dadurch gekennzeichnet, daß die Verbindung der Formel I in Konzentrationen von 0,01 bis 20 Gew.-% eingesetzt wird.

6. Flüssigkristallmischungen, gekennzeichnet durch einen Gehalt von 0.01 bis 20 Gew.-% an mindestens einer Verbindung der Formel I nach Anspruch 1.

7. Orientierungsschicht, gekennzeichnet durch einen Gehalt von 0.01 bis 20 Gew.-% an mindestens einer Verbindung der Formel I nach Anspruch 1.

8. Elektrooptisches Schalt- und Anzeigeelement, enthaltend die Flüssigkristallmischung und/oder die Orientierungsschicht nach Anspruch 6 oder 7.

## Claims

1. A compound of the formula I 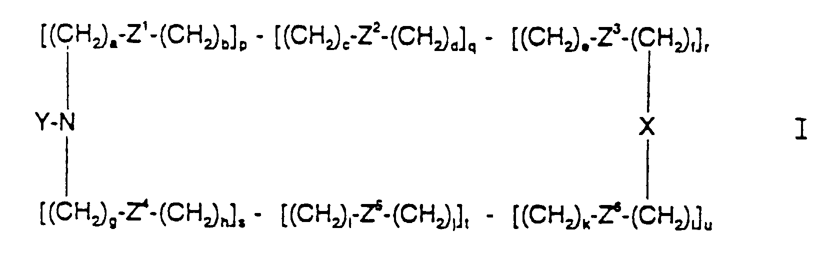 in which
- Z¹, Z², Z³, Z⁴, Z⁵ and Z⁶ are identical or different and are the -O- or -S- group, or are a single bond, but where at least 3 of the Z groups must be present in the formula I;
- Y is the B-(CH₂)ₘ-A- group, in which
A is 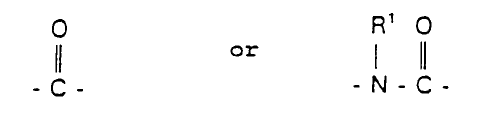
and B is 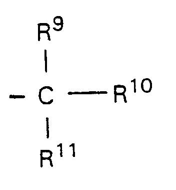
where R¹ is C₁-C₁₈-alkyl or phenyl, which may be substituted by 1 to 3 C₁-C₄-alkyl or -alkoxy groups, and
R⁹, R¹⁰ and R¹¹ are identical or different and are branched or unbranched alkyl having 1 to 6 carbon atoms, or together with the carrying carbon atom form a cyclic or polycyclic system having 2 to 12 rings;
- m is the number 0 or 1,
- X is N-Y, -O- or Z¹ to Z⁶,
- a, b, c, d, e, f, g, h, i, j, k and l are a number from 0 to 3, where the sum of a + b + c + d + e + f + g + h + i + j + k + l corresponds to from 8 to 16 carbon atoms, with the proviso that a, f, g and/or l cannot be zero, and that Z¹, Z² and Z³, and Z⁴, Z⁵ and Z⁶ are not directly adjacent; and
- p, q, r, s, t and u are 0 or 1, where the sum of p, q, r, s, t and u is from 2 to 6;
a, b, c, d, e, f, g, h, i, j, k, l and p, q, r, s, t and u must be selected so that ring sizes of from 12 to 24 arise.

2. A process for the stabilization of complex ligands, which comprises derivatizing the complex ligands by means of bulky secondary or tertiary carboxylic acids and/or sulfonic acids.

3. The use of a compound of the formula I as claimed in claim 1 as an additive to liquid-crystal mixtures.

4. The use of a compound of the formula I as claimed in claim 1 as an additive to alignment layers.

5. The use as claimed in claim 3 or 4, wherein the compound of the formula I is employed in a concentration of from 0.01 to 20% by weight.

6. A liquid-crystal mixture which contains from 0.01 to 20% by weight of at least one compound of the formula I as claimed in claim 1.

7. An alignment layer which contains from 0.01 to 20% by weight of at least one compound of the formula I as claimed in claim 1.

8. An electro-optical switching and display element containing a liquid-crystal mixture and/or an alignment layer as claimed in claim 6 or 7.

## Revendications

1. Composé de formule générale I 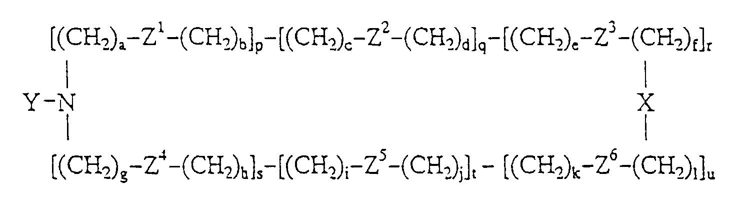 dans laquelle
- Z¹, Z², Z³, Z⁴, Z⁵, Z⁶ sont identiques ou différents et représentent le groupe -O- ou -S- ou une liaison simple, au moins 3 des groupes Z devant cependant être contenus dans la formule I;
- Y représente le groupe B-(CH₂)ₘ-A- dans lequel A est 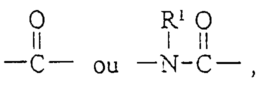 et B est 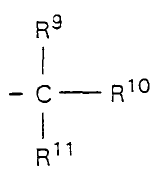 R¹ étant un groupe alkyle en C₁-C₈ ou phényle qui peut être substitué par 1 à 3 groupes alkyle ou alcoxy en C₁-C₃, et
R⁹, R¹⁰, R¹¹ sont identiques ou différents et représentent un groupe alkyle linéaire ou ramifié de 1 à 6 atomes de carbone, ou forment ensemble avec l'atome de carbone auquel ils sont liés un système cyclique ou polycyclique de 2 à 12 cycles;
- m représente le chiffre 0 ou 1,
- X est N-Y, -O- ou Z¹ à Z⁶,
- a, b, c, d, e, f, g, h, i, j, k, l sont des chiffres de 0 à 3, la somme a + b + c + d + e + f + g + h + i + j + k + l correspondant à 8 à 16 atomes de carbone, à condition que a, f, g et/ou l ne soient pas nuls et que Z¹, Z², Z³ et Z⁴, Z⁵, Z⁶ ne soient pas directement contigus; et
- p, q, r, s, t, u sont égaux à 0 ou 1, la somme de p, q, r, s, t et u étant comprise entre 2 et 6; et
il faut choisir a, b, c, d, e, f, g, h, i, j, k, l et p, q, r, s, t, u de façon à obtenir des cycles de 12 à 24 chaînons.

2. Procédé de stabilisation de ligands de complexes, caractérisé en ce que l'on forme des dérivés des ligands de complexes avec des acides carboxyliques et/ou des acides sulfoniques secondaires ou tertiaires encombrés.

3. Utilisation des composés de formule générale I selon la revendication 1 comme additifs pour des mélanges de cristaux liquides.

4. Utilisation du composé de formule générale I selon la revendication 1 comme additif dans des couches d'orientation.

5. Utilisation selon la revendication 3 ou 4, caractérisée en ce que l'on utilise le composé de formule I à des concentrations de 0,01 à 20 % en masse.

6. Mélanges de cristaux liquides, caractérisés par une teneur de 0,01 à 20 % en masse en au moins un composé de formule I selon la revendication 1.

7. Couche d'orientation, caractérisée par une teneur de 0,01 à 20 % en masse en au moins un composé de formule I selon la revendication 1.

8. Elément de circuit et de visualisation électro-optique, contenant le mélange de cristaux liquides et/ou la couche d'orientation selon la revendication 6 ou 7.
